# EUROPÄISCHE PATENTSCHRIFT

(11) **EP 2 148 890 B1**
(45) Veröffentlichungstag und Bekanntmachung des Hinweises auf die Patenterteilung: **19.08.2015**
(21) Anmeldenummer: 08759877.7
(22) Anmeldetag: 21.05.2008
(51) Int. Cl.: C08B 31/18

(54) **WASSERLÖSLICHE EISEN-KOHLENHYDRATDERIVAT-KOMPLEXE, DEREN HERSTELLUNG UND DIESE ENTHALTENDE ARZNEIMITTEL**
WATER-SOLUBLE IRON-CARBOHYDRATE DERIVATIVE COMPLEXES, PREPARATION THEREOF AND MEDICAMENTS COMPRISING THEM
COMPLEXES DE FER ET DE DÉRIVÉS D'HYDRATES DE CARBONE SOLUBLES DANS L'EAU, LEUR FABRICATION ET MÉDICAMENTS LES CONTENANT

(30) Priorität: 29.05.2007 EP 07109081
(43) Veröffentlichungstag der Anmeldung: 03.02.2010
(73) Patentinhaber: VIFOR (INTERNATIONAL) AG, 9001 St. Gallen (CH)
(72) Erfinder: REIM, Stefan, CH-9014 St. Gallen (CH); PHILIPP, Erik, CH-9303 Wittenbach (CH); FUNK, Felix, CH-8404 Winterthur (CH); MÜLLER, Hans-Martin, CH-9032 Engelburg (CH); GEISSER, Peter, CH-9014 St. Gallen (CH)
(74) Vertreter: Gille Hrabal
(86) Internationale Anmeldenummer: PCT/EP2008/056276
(87) Internationale Veröffentlichungsnummer: WO 2008/145586

(56) Entgegenhaltungen:
- WO-A-2004/037865
- WO-A-2006/111802

## Beschreibung

Gegenstand der vorliegenden Erfindung sind wasserlösliche Eisen-Kohlenhydratderivat-Komplexe, die zur Therapie von Eisenmangelzuständen geeignet sind, sowie deren Herstellung, diese enthaltende Arzneimittel und deren Verwendung bei der Prophylaxe oder Therapie von Eisenmangelzuständen. Die Arzneimittel sind insbesondere zur parenteralen Anwendung geeignet.

Durch Eisenmangel bedingte Anämien können durch Verabreichung von eisenhaltigen Arzneimitteln therapiert oder prophylaktisch behandelt werden. Hierzu ist der Einsatz von Eisen-Kohlenhydrat-Komplexen bekannt. Ein in der Praxis häufig erfolgreich angewandtes Präparat basiert auf einem wasserlöslichen Eisen(III)-Hydroxid-Saccharose-Komplex (Danielson, Salmonson, Derendorf, Geisser, Drug Res., Vol. 46 : 615 - 621, 1996). Im Stand der Technik werden zur parenteralen Verabreichung auch Eisen-Dextran-Komplexe sowie Komplexe auf der Basis schwer zugänglicher Pullulane (WO 02/46241), die unter Druck bei hohen Temperaturen und unter Einbeziehung von Hydrierschritten hergestellt werden müssen, beschrieben. Weitere Eisen-Kohlenhydrat-Komplexe sind zur oralen Verabreichung geläufig.

WO 2004/037865 der Anmelderin offenbart ein bevorzugt parenteral verabreichbares Eisenpräparat, das sich vergleichsweise einfach sterilisieren lässt; die vorher bekannten auf Saccharose bzw. Dextran basierenden parenteral verabreichbaren Präparate waren nämlich nur bei Temperaturen bis zu 100°C stabil, wodurch die Sterilisation erschwert wurde. Das Präparat weist eine verringerte Toxizität auf und birgt eine vermindertes Risiko für gefährliche durch Dextran induzierbare anaphylaktische Schocks. Durch die hohe Komplexstabilität wird eine hohe Applikationsdosis bzw. eine hohe Applikationsgeschwindigkeit ermöglicht. Das Eisenpräparat ist aus einfach erhältlichen Ausgangsprodukten ohne besonderen Aufwand herstellbar. Offenbart werden insbesondere wasserlösliche Eisen(III)-Kohlenhydrat-Komplexe auf der Basis der Oxidationsprodukte von Maltodextrinen sowie ein Verfahren zu deren Herstellung. Diese Eisen(III)-Kohlenhydrat-Komplexe sind erhältlich aus einer wässrigen Eisen(III)-Salzlösung und einer wässrigen Lösung des Produktes der Oxidation von einem oder mehreren Maltodextrinen mit einer wässrigen Hypochloritlösung bei einem alkalischen pH-Wert von z.B. 8 bis 12, wobei beim Einsatz von einem Maltodextrin dessen Dextrose-Äquivalent bei 5 bis 20 und beim Einsatz eines Gemisches aus mehreren Maltodextrinen das Dextrose-Äquivalent des Gemisches bei 5 bis 20 und das Dextrose-Äquivalent der am Gemisch beteiligten einzelnen Maltodextrine bei 2 bis 40 liegt.

T. Nakano et al., Nahrung/Food 47 (2002) No.4, S. 274-278 beschreibt ein Verfahren zur Phosphorylierung von u.a. Dextrin durch trockenes Erhitzen in Gegenwart von Phosphat. Phosphoryllerungsgrade des Dextrins von 1,07 %, 2,42 % und 3,2 % werden genannt, die in Abhängigkeit von Temperatur und Feuchtigkeit des Dextrins erhalten werden. Das erhaltene phosphorylierte Produkt wird auf seine Fähigkeit zur Solubilisierung von Phosphat untersucht. Die Möglichkeit, Caseinphosphopeptid als Calciumphosphat-Absorptionsverstärkendes Mittel durch phosphoryliertes.Dextrin zu ersetzen wird diskutiert. In dem genannten Dokument werden auch weitere Synthesemöglichkeiten für phosphorylierte Dextrine genannt, insbesondere Trocknen mit einer phosphathaltigen Lösung oder Trockenphosphorylierung mit Orthophosphat unter Erwärmen und Vakuum.

M.Z. Sitohy et al., Starch/Stärke 53 (2001), 317-322 beschreiben die Phosphorylierung von Stärke durch Mischen mit einer Lösung von Mononatrium- und Dinatriumphosphat, Abfiltrieren, Trocknen, Pulverisieren und anschließendes Erhitzen. Das phosphorylierte Produkt wird auf seine Hydrolysestabiltät bei saurer und enzymatischer Hydrolyse untersucht, und es wird die Verwendung im Gemisch mit Polyacrylat und Harnstoff in biologisch abbaubaren Kunststoffen vorgeschlagen.

US 4,841,040 beschreibt die Herstellung von phosphorylierten Dextrinen mit einem Molekulargewicht von 1.500 bis 40.000 Dalton und einem Substitutionsgrad von 0,30 bis 0,96 und deren Verwendung als Dispergiermittel für wässerige Aufschlämmungen von Mineralien und anorganischen Pigmenten mit hohem Festkörperanteil, als Ersatz für Gummi arabicum in Gummierungs- und Tintenlösungen für die Lithographie und als Bohrflüssigkeitszusatz. Der Substitutionsgrad ist dabei definiert durch das molare Verhältnis der derivatisierten Anhydroglucoseeinheiten zur Gesamtmenge der Anhydroglucoseeinheiten innerhalb eines Moleküls. Dieser wird im Folgenden molarer Substitutionsgrad (MS) genannt. Die phosphorylierten Dextrine werden erhalten durch Oxidation und Depolymerisation von Stärke durch Reaktion mit Natriumhypochlorit in alkalischen Medium und anschließende oder vorherige Phosphorylierung z.B. mit Phosphorsäure, Phosphorpentachlorid, Phosphorylchlorid oder polymeren Natriumorthophosphaten, insbesondere Natriumtrimetaphosphat.

CH-544 779 beschreibt ein Verfahren zur Herstellung von phosphorylierten Dextrinen durch Erhitzen eines Gemisches aus Stärke und einer Phosphorsäurelösung bei einem pH von weniger als 5 unter vermindertem Sauerstoffgehalt und dann weiteres Erhitzen in einer zweiten Stufe unter noch geringerem Sauerstoffgehalt bis zur Kondensation der Phosphorverbindung mit dem Stärkeprodukt, und anschließendes Kühlen unter vermindertem Sauerstoffgehalt. Das erhaltene Dextrinphosphat zeigt eine sehr hohe Wasserlöslichkeit. Die Verwendbarkeit als Oberflächenleim für Papiere, und zur Herstellung von Klebemitteln wird ebenfalls genannt.

WO 2006/082043 beschreibt in der Einleitung einige Verfahren zur Herstellung von Stärkephosphaten, z.B. nach dem Neukom-Verfahren (US 2,884,412} durch Suspendieren in wässriger Alkaliphosphatlösung, Filtrieren, Trocknen und Tempern bei Temperaturen um 140 °C, in einem homogenen Verfahren mit Tetrapolyphosphorsäure in Gegenwart von Tributylamin in Dimethylformamid (Towle et al., Methods Carbohydr. Chem. 6, (1972), 408-410) oder heterogen in einem Slurry-Verfahren in Benzol mit Phosphorsäureanhydrid (Tomasik et al., Starch/Stärke 43 (1991), 66-69). Das Dokument selbst schlägt ein Verfahren zur Herstellung von hochsubstituierten Stärkephosphaten vor, bei dem Stärke in einem Gemisch aus Phosphatierungsmittel (insbesondere Phosphatsalze oder Harnstoffphosphat) und Wasser sowie, falls das Phosphatierungsmittel frei ist von Harnstoff, Harnstoff gelöst wird, das Wasser entfernt wird und anschließend eine thermische Umsetzung zum Stärkephosphat erfolgt. Das erhaltene Stärkephosphat weist einen Substitutionsgrad an Phosphatgruppen von 0,01 bis 2,0 auf, sowie einen sehr geringen Gehalt an Carbamatgruppen. Die Verwendung der erhaltenen Stärkephosphate als Additiv in mineralischen oder dispersionsgebundenen Baustoffsystemen, als Additiv in der Pharmazie und Kosmetik, als anionische Komponente für Polyelektrolytkomplexe sowie als Trägermaterial wird vorgeschlagen.

US 3,732,207 offenbart die Herstellung von Dextrinestern mit organischen dibasischen Säureanhydriden, insbesondere Bernsteinsäureanhydrid oder Maleinsäureanhydrid, durch Erhitzen von Stärke oder Dextrin mit einem Restfeuchtigkeitsgehalt von etwa 3 % in Gegenwart des organischen Säureanhydrids in saurer Umgebung. Es wird ein Dextrinester mit einem molaren Substitutionsgrad von 0,02 bis 0,04 erhalten.

US 4,100,342 beschreibt die Herstellung von Dextrinestern durch Umsetzung von Dextrin mit Säureanhydriden von nichtaromatischen Carbonsäuren mit 2 bis 4 Carbonsäureeinheiten in Essigsäure in Gegenwart eines tertiären Amins als Katalysator und die Verwendung der erhaltenen Dextrinester als biologisch abbaubare Komponenten zur Erhöhung der Reinigungswirkung von Detergenzien.

WO 2004/064850 und WO 92/04904 offenbaren Dextrinsulfate und deren Verwendung, entweder allein oder in Kombination mit einem bakteriostatischen Mittel, als antivirale Zusammensetzung, insbesondere zur Behandlung von HIV und anderen sexuell übertragbaren Erkrankungen. Die Dextrinsulfate mit einem Substitutionsgrad von bis zu 2 Sulfatgruppen pro Glucoseeinheit werden hergestellt durch Hydrolyse von Stärke und anschließende Sulfatierung. Mit Trimethylamin/Schwefeltrioxid-Komplex in wässrigem alkalischem Medium erhält man hauptsächlich das 2-Sulfat, mit Cyclaminsäure in Dimethylformamid das 6-Sulfat und durch Acetylierung, anschließende Sulfatierung mit Trimethylamin/Schwefeltrioxid-Komplex in Dimethylformamid und schließlich Entfernung der Acetylgruppe mit wässeriger Natronlauge erhält man das 3-Sulfat. Die Wirkung der Dextrinsulfate gegen HIV sowie ihre antilipidämische Wirkung sind ebenfalls in diesen Dokumenten offenbart.

Keines der genannten Dokumente beschreibt jedoch die Bildung von Eisenkomplexen mit den erhaltenen Dextrinderivaten.

Aufgabe der vorliegenden Erfindung ist daher die Bereitstellung von neuen Eisen-Kohlenhydrat-Komplexen, die zur Behandlung von Eisenmangel-Anämie geeignet sind.

Die Aufgabe wird gelöst durch die Komplexe gemäß Anspruch 1. Bevorzugte Ausführungsformen der Komplexe sind in den Ansprüchen 2 und 3 definiert.

Die erfindungsgemäßen Komplexe werden durch das in den Ansprüchen 4 bis 10 definierte Verfahren erhalten.

Als Ausgangsprodukt werden erfindungsgemäß Maltodextrine verwendet. Diese sind leicht zugängliche Ausgangsprodukte, die im Handel erhältlich sind.

Zur Herstellung der Liganden der erfindungsgemäßen Komplexe werden die Maltodextrine zunächst in wässriger Lösung mit Hypochloritlösung oxidiert. Dieses Verfahren ist bereits in WO 2004/037865 beschrieben, deren Offenbarung hier vollinhaltlich durch Bezugnahme aufgenommen wird.

Geeignet sind beispielsweise Lösungen von Alkalihypochloriten, wie Natriumhypochloritlösung. Es können handelsübliche Lösungen eingesetzt werden. Die Konzentrationen der Hypochlorit-Lösungen liegen beispielsweise bei mindestens 13 Gew.-%, bevorzugt in der Größenordnung von 14 bis 16 Gew.-% jeweils berechnet als aktives Chlor. Die Lösungen werden bevorzugt in einer derartigen Menge eingesetzt, dass etwa 80 bis 100 %, bevorzugt etwa 90 % einer Aldehydgruppe pro Maltodextrinmolekül oxidiert werden. Auf diese Weise wird das durch die Glucoseanteile der Maltodextrinmoleküle bedingte Reduktionsvermögen auf etwa 20 % oder darunter, bevorzugt 10 % oder darunter verringert.

Die Oxidation erfolgt in alkalischer Lösung, beispielsweise bei pH-Werten von 8 bis 12, z.B. 9 bis 11. Zur Oxidation kann beispielsweise bei Temperaturen in der Größenordnung von 15 bis 40°C, bevorzugt 20 bis 35°C gearbeitet werden. Die Reaktionszeiten liegen beispielsweise in der Größenordnung von 10 Minuten bis 4 Stunden, z.B. 1 bis 1,5 Stunden.

Durch die beschriebene Verfahrensweise wird der Grad der Depolymerisation der eingesetzten Maltodextrine gering gehalten. Ohne eine bindende Theorie abzugeben, wird angenommen, dass die Oxidation vorwiegend an der endständigen Aldehydgruppe (bzw. Halbacetalgruppe) der Maltodextrinmoleküle erfolgt. Dieser Syntheseschritt wird im folgenden vereinfachend als "C₁-Oxidation" bezeichnet, ohne dass jedoch eine Bindung an diese Bezeichnung beabsichtigt ist.

Es ist auch möglich, die Oxidationsreaktion der Maltodextrine zu katalysieren. Geeignet hierzu ist der Zusatz von Bromidionen, z.B. in der Form von Alkalibromiden, beispielsweise Natriumbromid. Die zugesetzte Menge an Bromid ist nicht kritisch. Sie wird möglichst gering gehalten, um ein möglichst leicht zu reinigendes Endprodukt (Fe-Komplex) zu erhalten. Es genügen katalytische Mengen. Wie erwähnt, ist der Zusatz von Bromid zwar möglich, aber nicht erforderlich.

Darüber hinaus ist es beispielsweise auch möglich, das bekannte ternäre Oxidationssystem Hypochlorit/Alkalibromid/2,2,6,6-Tetramethylpiperidin-1-oxyl (TEMPO) zur Oxidation der Maltodextrine zu verwenden. Die Verfahrensweise, Maltodextrine unter Katalyse von Alkalibromiden bzw. mit dem ternären TEMPO-System zu oxidieren, wird beispielsweise von Thaburet et al. in Carbohydrate Research 330 (2001) 21 - 29 beschrieben; die dort beschriebene Verfahrensweise ist erfindungsgemäß anwendbar.

Die Aufarbeitung und Isolierung der oxidierten Maltodextrine erfolgt durch Einstellung der Reaktionslösung auf einen etwa neutralen pH durch geeignete Säuren oder Puffer wie z.B. Salzsäure, Schwefelsäure oder Essigsäure.

Anschließend kann das oxidierte Reaktionsprodukt durch Zusatz eines geeigneten Lösungsmittels, in dem es im wesentlichen unlöslich ist, ausgefällt werden. Als Lösungsmittel kommt z.B. Ethanol in Frage, das bevorzugt in einer Konzentration von 80 bis 95 Gew.-% eingesetzt wird, besonders bevorzugt 90 bis 94 Gew.-%, in einem Volumenverhältnis Ethanol : Reaktionslösung von ca. 1:5 bis 1:10, bevorzugt 1:5 bis 1:8. Weiter sind als Fällungslösungsmittel Methanol, Propanol oder Aceton geeignet. Der Niederschlag wird anschließend auf übliche Art und Weise abfiltriert und getrocknet.

Alternativ kann die Reaktionslösung mittels Dialyse oder Membranfiltration aufgereinigt und das Produkt durch Lyophilisation oder Sprühtrocknung erhalten werden.

Das C₁-oxidierte Maltodextrin kann aber auch ohne Isolierung direkt im nachfolgenden Derivatisierungsschritt eingesetzt werden.

Die anschließende Derivatisierung der erhaltenen C₁-oxidierten Produkte erfolgt durch übliche, dem Fachmann bekannte Verfahren der Derivatisierung von Zuckern, z.B. durch Oxidation, Veresterung mit einfach- oder mehrfach funktionellen anorganischen oder organischen Säuren oder Säurederivaten, Carboxyalkylierung, Addition von organischen Isocyanaten, Veretherung, Amidierung, Anhydridbildung etc.

So kann z.B. mit organischen Säuren oder Säurederivaten eine Veresterung erfolgen. Es können beliebige, dem Fachmann bekannte Carbonsäuren oder reaktive Carbonsäurederivate für die Veresterung eingesetzt werden, bevorzugt Säurechloride, -anhydride oder -bromide. Bevorzugt werden C₁-C₆-Carbonsäurederivate für die Veresterung eingesetzt, besonders bevorzugt Acetanhydrid. Die Veresterung erfolgt unter üblichen Reaktionsbedingungen, z.B. in wässriger Lösung oder in einem geeigneten Lösungsmittel wie z.B. Formamid, Dimethylformamid, Dimethlysulfoxid oder Essigsäure. Die Reaktion in wässriger Lösung kann z.B. bei leicht basischem pH von ca. 7,5 bis 10, bevorzugt 8 bis 9,5 (der pH kann mit beliebigen Basen eingestellt und während der Reaktion konstant gehalten werden, z.B. Alkali- oder Erdalkalihydroxide wie Natrium- oder Kaliumhydroxid sowie Alkali- oder Erdalkalicarbonate) durch Zugabe des reaktiven Carbonsäurederivats, z.B. Acetylchlorid oder Acetanhydrid erfolgen. Bei Verwendung eines anderen Lösungsmittels werden dieselben Reagenzien verwendet und die Reaktionsbedingungen geeignet ausgewählt. Die Umsetzung kann in den genannten Lösungsmitteln bei Raumtemperatur, unter Kühlen oder Erwärmen stattfinden. Die Reaktionszeit beträgt z.B. 0,5 bis 2 Stunden, bevorzugt 0,75 bis 1,5 Stunden. Die Aufarbeitung erfolgt wie bei der C₁-Oxidation beschrieben durch Ausfällung, Abfiltrieren und Trocknen.

Auf dieselbe Weise kann auch eine Veresterung mit mehrbasigen organischen Carbonsäuren erfolgen, z.B. die Herstellung von Bernsteinsäure-, Maleinsäure-, Fumarsäure-, Glutarsäure- oder Adipinsäureestern, wobei die zweite Carboxylgruppe des Esters entweder frei oder als Alkylester vorliegen kann. Zur Herstellung sind die Anhydride, Mischanhydride, -chloride oder - bromide oder sonstige reaktive Derivate der mehrbasigen Carbonsäuren geeignet, so insbesondere Bernsteinsäureanhydrid, Maleinsäureanhydrid, Glutarsäureanhydrid, Adipinsäureanhydrid oder Fumarsäureedichlorid. Die Umsetzung und Aufarbeitung erfolgt wie bei der Veresterung beschreiben. Besonders bevorzugt ist die Veresterung mit Bernsteinsäureanhydrid zum Succinyl-Maltodextrin.

Ebenfalls können die C₁-oxidierten Maltodextrine zu Carboxyalkylderivaten umgesetzt werden. Als Reagenz sind dem Fachmann bekannte Carboxyalkylhalogenide geeignet, z.B. Halogencarbonsäuren wie Chlor- oder Bromcarbonsäuren bzw. deren Natrium- oder Kaliumsalze, z.B. in beliebiger Position halogenierte C₁-C₆-Carbonsäuren wie z.B. α- oder β-Brompropionsäure oder besonders bevorzugt Chlor- oder Bromessigsäure.

Die Umsetzung erfolgt auf dem Fachmann bekannt Weise z.B. in wässriger Lösung oder in einem geeigneten Lösungsmittel wie z.B. Formamid, Dimethylformamid, Dimethylsulfoxid oder Essigsäure. In wässriger Lösung erfolgt die Umsetzung z.B. bei basischen pH (pH 11 bis 14, bevorzugt ca. 12,5 bis 14, Einstellung mit beliebigen Basen, z.B. NaOH). Bei Verwendung eines anderen Lösungsmittels werden dieselben Reagenzien verwendet und die Reaktionsbedingungen geeignet ausgewählt. Die Umsetzung kann in den genannten Lösungsmitteln bei Raumtemperatur, unter Kühlen oder Erwärmen während z. B. 0,5 bis 5 Stunden, bevorzugt ca. 2,5 bis 3,5 Stunden stattfinden. Die Aufarbeitung und Isolierung erfolgt wie bei der Veresterung beschrieben.

Die Veresterung mit reaktiven Derivaten anorganischer Säuren, z.B. die Sulfatierung oder Phosphatierung, erfolgt ebenfalls nach dem Fachmann bekannten Verfahren.

Die Sulfatierung erfolgt z.B. in wässriger Lösung oder einem geeigneten Lösungsmittel wie z. B. Formamid, Dimethylformamid, Dimethylsulfoxid oder Essigsäure mit einem geeigneten Sulfatierungsreagens, z.B. SO₃-Trimethylamin-Komplex oder Cyclaminsäure bei Raumtemperatur, unter Kühlen oder Erwärmen , bevorzugt z.B. bei 30 °C während einer geeigneten Zeit, z.B. 15 Minuten bis 2 Stunden, bevorzugt ca. 30 Minuten. Anschließend wird bei Verwendung von Wasser als Lösungsmittel der pH der Reaktionslösung stark basisch gestellt (z. B. auf pH 12 - 13) und die Lösung weiter bei geeigneter Temperatur, z.B. 30 °C, gerührt. Nach Ansäuern auf pH 9,5 bis 11, bevorzugt ca. 10,5, mit einer geeigneten Säure oder einem Puffer, wie z.B. HCl erfolgt die Ausfällung und Isolierung wie bei der C₁-Oxidation beschrieben.

Die Phosphatierung erfolgt nach einem beliebigen den Fachleuten bekannten Verfahren. Eine Möglichkeit besteht darin, dass Dextrin mit dem Phosphatierungsreagens in Wasser zu lösen und einen pH-Wert von 2 - 6, bevorzugt etwa 3, einzustellen. Als Phosphatierungsreagens kommen alle bekannten Reagenzien In Frage, bevorzugt wird ein Gemisch Natriumdihydrogenphosphat/Dinatriumhydrogenphosphat im molaren Verhältnis 1 : 0.5 - 1 : 2.5, z. B. 1 : 1.8, eingesetzt. Die Reaktionslösung kann z. B. mit Ethanol, Methanol oder Aceton ausgefällt und der Niederschlag isoliert und getrocknet werden, oder die Reaktionslösung wird zur Trockne eingedampft, z.B. in einem Rotationsverdampfer, und weiter getrocknet, bevorzugt bei erhöhter Temperatur und Im Vakuum. Nach Mahlen wird das Produkt trocken erhitzt, z.B. auf 1 20 bis 180 °C, bevorzugt 150 bis 170 °C, bevorzugt im Vakuum, dann erneut gemahlen und anschließend in Wasser oder einem geeigneten Lösungsmittel gelöst, bevorzugt bei erhöhter Temperatur von z.B. 50 °C. Anschließend werden unlösliche Rückstände abgetrennt, z.B. durch Zentrifugieren oder Filtrieren, und die erhaltene Lösung zur Entfernung von freiem Orthophosphat durch Membranfiltration gereinigt. Die Filtration kann durch IR-Spektroskopie oder Leitfähigkeitsmessung verfolgt werden. Nachdem alles Orthophosphat entfernt ist, wird die Lösung am Rotationsverdampfer aufkonzentriert und dann ausgefällt und isoliert wie bei der Veresterung beschrieben.

C₂/C₃-oxidierte Derivate können durch dem Fachmann bekannte Oxidation des C₁-oxidierten Maltodextrins mit einem geeigneten Oxidationsmittel wie z. B. NaOCl oder NalO₄/NaOCl₂ erhalten werden. Die Oxidation erfolgt z.B. in wässriger Lösung oder in einem geeigneten Lösungsmittel wie Dimethylformamid, Formamid, Dimethylsulfoxid oder Essigsäure bei Raumtemperatur, unter Erwärmung oder Kühlung. Bei Verwendung von Wasser als Lösungsmittel erfolgt die Reaktion bei leicht basischem, konstantem pH von 7,5 bis 9,5, bevorzugt 8,5 bis 9,0, z.B. durch Natriumhypochlorit bei ca. 50 °C. Anschließend wird der pH neutral eingestellt, z.B. durch Zugabe von HCl, und das Produkt anschließend ausgefällt und isoliert wie bei der Veresterung beschrieben.

Durch Verwendung unterschiedlicher Mengen der jeweiligen Reagenzien zur Derivatisierung können unterschiedliche molare Substitutionsgrade erzielt werden. Der molare Substitutionsgrad ist dabei definiert durch das molare Verhältnis der derivatisierten Anhydroglucoseeinheiten zur Gesamtmenge der Anhydroglucoseeinheiten innerhalb eines Moleküls.

Die Produkte werden durch IR-Spektroskopie untersucht. So kann qualitativ festgestellt werden, ob die gewünschte funktionelle Gruppe ins Maltodextrin eingeführt wurde. Die Einführung einer Carboxylgruppe, z.B. einer Acetylgruppe, Succinylgruppe oder Carboxymethylgruppe, kann durch Zunahme der Bande bei 1740 cm⁻¹ im IR-Spektrum verfolgt werden (C=O-Valenzschwingung von COOR). Die erfolgte C₂/C₃-Oxidation kann durch Zunahme der Bande bei 1640 cm⁻¹ verfolgt werden (C=O-Valenzschwingung von COO⁻).Die Einführung einer Sulfatgruppe kann durch Anstieg der Banden bei 1260 und 830 cm⁻¹ bestätigt werden (Valenzschwingungen von SO₄²⁻). Die Einführung einer Phosphatgruppe kann auch durch ³¹P-NMR-Spektroskopie qualitativ bestätigt werden. Polymer-gebundenes Monophosphat erscheint in Form breiter Signale bei ca. 0 bis 2 ppm, während freies Monophosphat ein scharfes Signal bei ca. 0,7 ppm zeigt.

Die quantitative Bestimmung des molaren Substitutionsgrads kann durch ¹H-NMR-Spektroskopie oder ¹³C-NMR-Spektroskopie erfolgen, indem die Intensität eines der eingeführten funktionellen Gruppe zugeordneten Signals ins Verhältnis gesetzt wird zur Intensität eines Signals des Maltodextrins, die durch die Derivatisierung nicht beeinflusst wird. Im Fall der Phosphatierung kann die quantitative Bestimmung des molaren Susbstitutionsgrads auch durch ICP-OES (Inductively Coupled Plasma-Optical Emission Spectroscopy, Gesamtphosphatgehalt) und Ionenchromatographie gekoppelt mit Leitfähigkeitsmessung (Gehalt an freiem Monophosphat) erfolgen.

Zur Herstellung der erfindungsgemäßen Komplexe werden die erhaltenen oxidierten derivatisierten Maltodextrine in wässriger Lösung mit einem Eisen(III)-Salz umgesetzt. Hierzu können die oxidierten derivatisierten Maltodextrine isoliert und erneut gelöst werden; die erhaltenen wässrigen Lösungen der oxidierten derivatisierten Maltodextrine können jedoch auch direkt zur Weiterverarbeitung mit wässrigen Eisen(III)-Lösungen verwendet werden.

Als Eisen(III)-Salze können wasserlösliche Salze anorganischer oder organischer Säuren oder Mischungen davon verwendet werden, wie Halogenide, z.B. Chlorid und Bromid, oder Sulfate. Bevorzugt werden physiologisch unbedenkliche Salze verwendet. Besonders bevorzugt wird eine wässrige Lösung von Eisen(III)-Chlorid verwendet.

Es hat sich gezeigt, dass sich die Anwesenheit von Chloridionen günstig auf die Komplexbildung auswirkt. Letztere können beispielsweise in der Form von wasserlöslichen Chloriden, wie Alkalimetallchloriden, z.B. Natriumchlorid, Kaliumchlorid oder Ammoniumchlorid, zugesetzt werden. Bevorzugt wird, wie erwähnt, das Eisen(III) in der Form des Chlorids eingesetzt.

Zur Umsetzung kann beispielsweise die wässrige Lösung des oxidierten Maltodextrins mit einer wässrigen Lösung des Eisen(III)-Salzes vermischt werden. Dabei wird bevorzugt so gearbeitet, dass der pH-Wert des Gemisches aus oxidiertem Maltodextrin und Eisen(III)-Salz beim und unmittelbar nach dem Vermischen zunächst stark sauer ist, bzw. so niedrig ist, dass keine Hydrolyse des Eisen(III)-Salzes auftritt, z.B. 2 oder darunter beträgt, um eine unerwünschte Ausfällung von Eisenhydroxiden zu vermeiden. Beim Einsatz von Eisen(III)-Chlorid ist im allgemeinen kein Säurezusatz erforderlich, da wässrige Lösungen von Eisen(III)-Chlorid selbst ausreichend sauer sein können. Nach dem erfolgten Vermischen kann der pH-Wert beispielsweise auf Werte in der Größenordnung von gleich oder größer als 5, beispielsweise bis zu 11, 12, 13 oder 14 angehoben werden. Das Anheben des pH-Wertes erfolgt bevorzugt langsam bzw. allmählich, was beispielsweise dadurch erfolgen kann, dass zunächst eine schwache Base zugesetzt wird, beispielsweise bis zu einem pH von etwa 3; anschließend kann dann mit einer stärkeren Base weiter neutralisiert werden. Als schwache Base kommen beispielsweise Alkali- oder Erdalkalicarbonate, -bicarbonate, wie Natrium- und Kaliumcarbonat oder -bicarbonat oder Ammoniak infrage. Starke Basen sind beispielsweise Alkali- oder Erdalkalihydroxide, wie Natrium-, Kalium-, Calcium- oder Magnesiumhydroxid.

Die Umsetzung kann durch Erwärmen begünstigt werden. Beispielsweise können Temperaturen in der Größenordnung von 15°C bis zur Siedetemperatur angewendet werden. Es ist bevorzugt, die Temperatur allmählich zu steigern. So kann beispielsweise zunächst auf etwa 15 bis 70°C erwärmt und allmählich bis zum Sieden gesteigert werden.

Die Reaktionszeiten liegen beispielsweise in der Größenordnung von 15 Minuten bis zu mehreren Stunden, z.B. 20 Minuten bis 4 Stunden, beispielsweise bei 25 bis 70 Minuten, z.B. 30 bis 60 Minuten.

Die Umsetzung kann im schwach sauren Bereich, beispielsweise bei pH-Werten in der Größenordnung von 5 bis 6, erfolgen. Es hat sich aber gezeigt, dass es zweckmäßig, wenn auch nicht erforderlich ist, den pH-Wert im Verlauf der Komplexbildung auf höhere Werte, bis zu 1 1, 12, 13 oder 14 anzuheben. Zur Fertigstellung der Reaktion kann der pH-Wert dann durch Säurezusatz weiter gesenkt werden, beispielsweise auf die genannte Größenordnung von 5 bis 6. Als Säuren können anorganische oder organische Säuren oder Gemische davon, insbesondere Halogenwasserstoffsäuren, wie Chlorwasserstoff bzw. wässrige Salzsäure eingesetzt werden.

Wie erwähnt, wird die Komplexbildung im allgemeinen durch Erwärmen begünstigt. Beispielsweise kann bei der bevorzugten Ausführungsform, bei der der pH-Wert im Verlauf der Umsetzung auf Bereiche von über 5 hinaus bis zu 11 oder 14 gesteigert wird, zunächst bei niedrigen Temperaturen in der Größenordnung von 15 bis 70°C, z.B. 40 bis 60°C, z.B. etwa 50°C gearbeitet werden, worauf nach erneuter Verringerung des pH-Wertes beispielsweise auf Werte in der Größenordnung von mindestens 5, allmählich auf Temperaturen über 50°C bis zur Siedetemperatur erwärmt wird.

Die Reaktionszeiten liegen in der Größenordnung von 15 Minuten bis zu mehreren Stunden und können je nach Reaktionstemperatur variieren. Bei der Durchführung des Verfahrens unter zwischenzeitlicher Anwendung von pH-Werten, die über 5 liegen, kann beispielsweise 15 bis 70 Minuten, z.B. 30 bis 60 Minuten bei dem erhöhten pH-Wert, beispielsweise bei Temperaturen bis zu 70°C gearbeitet werden, worauf die Reaktion nach Absenken des pH-Wertes auf den Größenordnungsbereich von mindestens 5, weitere 15 bis 70 Minuten, z.B. 30 bis 60 Minuten bei Temperaturen bis zu beispielsweise 70°C und gegebenenfalls weitere 15 bis 70 Minuten, z.B. 30 bis 60 Minuten bei höheren Temperaturen bis zum Siedepunkt durchgeführt werden kann.

Nach erfolgter Umsetzung kann die erhaltene Lösung beispielsweise auf Raumtemperatur abgekühlt und gegebenenfalls verdünnt und gegebenenfalls filtriert werden. Nach dem Abkühlen kann der pH-Wert durch Zugabe von Säure oder Base auf den Neutralpunkt oder leicht darunter, beispielsweise auf Werte von 5 bis 7 eingestellt werden. Als Säuren oder Basen können beispielsweise die vorstehend zur Umsetzung genannten verwendet werden. Die erhaltenen Lösungen werden gereinigt und können direkt zur Herstellung von Arzneimitteln verwendet werden. Es ist aber auch möglich, die Eisen(III)-Komplexe aus der Lösung zu isolieren, beispielsweise durch Ausfällen mit einem Alkohol, wie einem Alkanol, beispielsweise Ethanol. Die Isolierung kann auch durch Sprühtrocknung erfolgen. Die Reinigung kann in üblicher Weise erfolgen, insbesondere zur Entfernung von Salzen. Dies kann z.B. durch Umkehrosmose erfolgen, wobei eine derartige Umkehrosmose z.B. vor der Sprühtrocknung oder vor dem direkten Einsatz in Arzneimitteln durchgeführt werden kann.

Die erhaltenen Eisen(III)-Kohlenhydrat-Komplexe weisen beispielsweise einen Eisengehalt von 10 bis 40 % Gew./Gew., insbesondere 20 bis 35 % Gew./Gew. auf. Sie sind gut wasserlöslich. Man kann daraus neutrale wässrige Lösungen mit beispielsweise 1 % Gew./Vol. bis 20 % Gew./Vol. Eisengehalt herstellen. Diese Lösungen lassen sich thermisch sterilisieren. Das gewichtsmittlere Molekulargewicht Mw der so erhaltenen Komplexe beträgt beispielsweise 80 kDa bis 800 kDα, bevorzugt 80 bis 650 kDa, besonders bevorzugt bis zu 350 kDa (bestimmt mittels Gelpermeationschromatographie, beispielsweise wie von Geisser et al. in Arzneim. Forsch/Drug Res. 42(II), 12, 1439 - 1452 (1992), Absatz 2.2.5. beschrieben).

Wie erwähnt, lassen sich aus den erfindungsgemäßen Komplexen wässrige Lösungen herstellen. Diese sind insbesondere zur parenteralen Applikation geeignet. Sie können jedoch auch oral oder topisch angewendet werden. Sie können bei hohen Temperaturen, z. B. bei 121°C und darüber sterilisiert werden, bei kurzen Kontaktzeiten von mindestens 15 Minuten unter Erreichen von Fₒ ≧ 15. F₀ ist dabei die Behandlungszeit in Minuten bei einer variablen Temperatur, die der Behandlungszeit in Minuten bei 121°C entspricht, berechnet für einen ideellen Mikroorganismus mit einem Temperaturkoeffizienten der mikrobiellen Zerstörung von 10. Bisher bekannte Präparate mussten teilweise bei Raumtemperatur steril filtriert und/oder teilweise mit Konservierungsmitteln, wie Benzylalkohol oder Phenol versetzt werden. Derartige Arbeitsschritte bzw. Zusätze sind erfindungsgemäß nicht nötig. Es ist möglich, die Lösungen der Komplexe beispielsweise in Ampullen abzufüllen. Beispielsweise lassen sich Lösungen von 1 bis 20 Gew.-%, beispielsweise 5 Gew.-% in Behälter, wie Ampullen oder Stechampullen (Vials) von beispielsweise 2 bis 100 ml, beispielsweise bis zu 50 ml abfüllen. Die Herstellung der parenteral verabreichbaren Lösungen kann in üblicher Weise, gegebenenfalls unter Mitverwendung von für parenterale Lösungen üblichen Zusätzen, erfolgen. Die Lösungen können so formuliert werden, dass sie als solche durch Injektion oder als Infusion, z.B. in Kochsalzlösung, verabreicht werden können. Zur oralen oder topischen Verabreichung können Präparate mit entsprechenden üblichen Exzipienten und Hilfsmitteln formuliert werden.

Einen weiteren Gegenstand der Erfindung bilden daher Arzneimittel, die insbesondere zur parenteralen, intravenösen, aber auch intramuskulären Verabreichung, sowie zur oralen oder topischen Verabreichung, geeignet sind und insbesondere für die Behandlung von Eisenmangelanämien Verwendung finden können. Ein weiterer Gegenstand der Erfindung betrifft daher auch die Verwendung der erfindungsgemäßen Eisen(III)-Kohlenhydratderivat-Komplexe zur Behandlung und Prophylaxe von Eisenmangelanämien bzw. zur Herstellung von Arzneimitteln zur insbesondere parenteralen Behandlung von Eisenmangelanämien. Die Arzneimittel sind zum Einsatz in der Human- und der Veterinärmedizin geeignet.

Erfindungsgemäß ist es erstmals möglich, Eisenkomplexe von MaltodextrinDerivaten herzustellen.

Gegenüber den aus WO 2004/037865 bekannten Eisen-Maltodextrin-Komplexen ermöglichen die erfindungsgemäßen Elsen-Maltodextrinderivat-Komplexe eine gezielte und feine Einstellung des Molekulargewichts über einen weiten Bereich hin zu höheren Molekulargewichten, was mit den bekannten Komplexen nicht möglich war.

Die Mehrzahl der Eisen-Maltodextrinderivat-Komplexe zeigt eine nahezu unveränderte Abbaukinetik (Θ = 0.5) gegenüber den aus WO 2004/037865 bekannten Eisen-Maltodextrin-Komplexen.

Die meisten derivatisierten Maltodextrinkomplexe zeigen eine erhöhte Stabilität gegenüber einem enzymatischen Abbau durch Amylase verglichen mit dem nicht derivatisierten Maltodextrin, was einen retardierten und gleichmässigen Abbau der erfindungsgemässen Eisen-Maltodextrinderivat-Komplexe im Körper fördern kann.

Die Eisenausbeuten der erfindungsgemäßen Komplexderivate reichen bis zu 100% (insbesondere bei den sulfatierten Komplexderivaten), verglichen mit 87 bis 93 % bei den bekannten Eisen-Maltodextrin-Komplexen, was einen wirtschaftlichen Vorteil für die Herstellung im kommerziellen Maßstab bedeutet.

### Beispiele

In der vorliegenden Beschreibung und den nachstehenden Beispielen werden die Dextrose-Äquivalente gravimetrisch bestimmt. Hierzu werden die Maltodextrine in wässriger Lösung mit Fehling'scher Lösung unter Sieden umgesetzt. Die Umsetzung erfolgt quantitativ, d.h. bis keine Entfärbung der Fehling'schen Lösung mehr auftritt. Das ausgefällte Kupfer(I)-Oxid wird bei 105°C bis zur Gewichtskonstanz getrocknet und gravimetrisch bestimmt. Aus den erhaltenen Werten wird der Glucosegehalt (Dextrose-Äquivalent) als % Gew./Gew. der Maltodextrin-Trockensubstanz berechnet. Es kann beispielsweise mit folgenden Lösungen gearbeitet werden: 25 ml Fehling'sche Lösung I, vermischt mit 25 ml Fehling'scher Lösung II; 10 ml wässrige Maltodextrinlösung (10 % Mol/Vol) (Fehling'sche Lösung I: 34,6 g Kupfer(II)-Sulfat gelöst in 500 ml Wasser; Fehling'sche Lösung II: 173 g Kaliumnatriumtartrat und 50 g Natriumhydroxid, gelöst in 400 ml Wasser).

Im folgenden wird erläutert, mit welchen Verfahren und Geräten die jeweiligen Eigenschaften der Maltodextrinderivate und Eisenkomplexe bestimmt wurden.
¹H-NMR: Bruker Avance-400, 400 MHz, Lösung in D₂O referenziert auf H₂O
¹³C-NMR: Bruker Avance-400, 100 MHz, Lösung in D₂O referenziert extern auf Trimethylsilyl-tetradeuteropropionsäure
³¹P-NMR: Bruker Avance-400, 162 MHz, Lösung in D₂O referenziert extern auf konz. H₃PO₄
IR: FT-IR Perkin Elmer 1 725x, KBr-Pressling
ICP-OES: Horiba Jobin Yvon Ultima 2, Probe gelöst in H₂O
IC: Metrohm 733 IC Separation Center (incl. Leitfähigkeits-Detektor), Probe gelöst in H₂O
GPC: Waters 515 HPLC Pumpe, Waters 2410 Refractive Index Detector, Probe gelöst in H₂O, Pullulan als Standard
Bestimmung von M_{w}: siehe GPC
Bestimmung von Mₙ: siehe GPC
Fe-Gehalt: Titrimetrische Bestimmung mit EDTA (z. B. Jander Jahr, Massanalyse 15. Auflage)
Abbaukinetik: P. Geisser, M. Baer, E. Schaub; Structure/Histotoxicity Relationship of Parenteral Iron Preparations; Arzneim.-Forsch./Drug Research 42 (II), 12, 1439 - 1452 (1992).
Analytik Jena Specord 205 Spektralphotometer, untersuchter Abbaugrad 50 % (Θ = 0.5)
Eisen-Ausbeute: Isolierte Fe-Menge in g/eingesetzte Fe-Menge in g

### Beispiel 1

### Herstellung von C₁-oxidiertem Maltodextrin

250 g Maltodextrin mit einem Dextroseäquivalent von 12 wurden in 750 ml Wasser gelöst. 1,4 g NaBr wurden zugegeben und 78,4 g NaOCl-Lösung (14 bis 16 Gew.-% aktives Chlor) wurden innerhalb von 30 Minuten zudosiert, wobei der pH bei 9,5 (±0,5) durch Zugabe von 30 Gew.-%iger NaOH konstant gehalten wurde. Dann wurde der pH mit HCl (20 Gew.-%) auf 7,0 eingestellt und das Produkt durch Zugabe von Ethanol (92 Gew.-%) in einem Volumenverhältnis 1:6 (Lösung : Ethanol) ausgefällt. Das Produkt wurde durch Abdekantieren der überstehenden Lösung isoliert und während 24 Stunden bei 50 °C und 125 mbar getrocknet.

### Beispiel 2

### Herstellung von C₁-oxidiertem Maltodextrin

100 g Maltodextrin (9,6 Dextrose-Äquivalente, gravimetrisch bestimmt) werden bei 25°C unter Rühren in 300 ml Wasser gelöst und durch Zugabe von 30 g Natriumhypochloritlösung (14 bis 16 Gew.-% aktives Chlor) bei pH 10 oxidiert und wie in Beispiel 1 isoliert und getrocknet.

### Beispiel 3

### Herstellung von C₁-oxidiertem Maltodextrin

Eine Mischung aus 45 g Maltodextrin (6,6 Dextrose-Äquivalente, gravimetrisch bestimmt) und 45 g Maltodextrin (14,0 Dextrose-Äquivalente, gravimetrisch bestimmt) wird bei 25°C unter Rühren in 300 ml Wasser gelöst und durch Zugabe von 25 g Natriumhypochloritlösung (14 bis 16 Gew.-% aktives Chlor) und 0,6 g Natriumbromid bei pH 10 oxidiert und wie in Beispiel 1 Isoliert und getrocknet.

### Beispiele 4 bis 7

### Acetylierung

200 g in Beispiel 1 erhaltenes Maltodextrin (1,23 Mol Anhydroglucose) wurden in 660 ml Wasser bei 25 °C gelöst, und der pH wurde mit 30 Gew.-%iger NaOH auf 8,5 eingestellt. Acetanhydrid wurde mit einer Rate von 1,7 ml/Min. in unterschiedlichen in Tabelle 1 gezeigten Mengen zugegeben, wobei der pH durch Zugabe von 30 Gew.-%iger NaOH konstant bei 8,5 (± 0,5) gehalten wurde. Die Lösung wurde während einer Stunde bei einem konstanten pH von 8,5 (± 0,5) gerührt und anschließend mit 20 Gew.-%iger HCl auf 7,0 eingestellt. Das Produkt wurde mit Ethanol (92 Gew.-%) im Volumenverhältnis 1:6 (Lösung : Ethanol) ausgefällt. Das Produkt wurde durch Abdekantieren der überstehenden Lösung isoliert und während 24 Stunden bei 50 °C und 1 25 mbar getrocknet.

Durch Variation der Menge an zugegebenem Acetanhydrid wurden unterschiedliche Acetylierungsgrade erhalten. Die Ergebnisse sind in Tabelle 1 dargestellt.

**Tabelle 1**

| Beispiel | Äquivalente Ac₂O (bezogen auf Anhydro-glucose) | Molarer Substitutionsgrad (¹H-NMR) | Ausbeute [%] (Mol isoliertes Produkt/Mol eingesetzte Anhydroglucose) |
|---|---|---|---|
| 4 | 1 | 0,84 | 24 |
| 5 | 0,67 | 0,61 | 65 |
| 6 | 0,33 | 0,31 | 69 |
| 7 | 0,16 | 0,14 | 74 |
| 1 | - | nicht derivatisiert | 84 |

Aufgrund der Acetylierung erhöht sich die Löslichkeit des Maltodextrinderivats in Ethanol, was in einer sinkenden Ausbeute mit steigendem Substistutionsgrad resultiert.

Der Acetylierungsgrad wurde qualitativ durch IR-Spektroskopie und quantitativ durch NMR-Spektroskopie bestimmt.

Durch IR-Spektroskopie kann die Acetylierung durch Zunahme der Bande bei 1 740 cm⁻¹ (C=O-Valenzschwingung von COOR) verfolgt werden. Der molare Acetylierungsgrad wurde durch ¹H-NMR-Spektroskopie durch das Verhältnis der Intensität des CH₃-Signals bei 2,0 - 2,3 ppm (Acetylgruppe) zur Intensität des Signals bei 3,0 - 4,5 ppm und 5 - 6 ppm (7 Protonen der Anhydroglucosegruppe) bestimmt.

### Beispiele 8 bis 11

### Succinylierung

200 g in Beispiel 1 erhaltenes C₁-oxidiertes Maltodextrin wurden in 655 ml Wasser gelöst. Der pH wurde mit 30 Gew.-%iger NaOH auf 8,5 eingestellt, und Bernsteinsäureanhydrid wurde portionsweise innerhalb einer Stunde bei 25 °C zugegeben, wobei der pH durch Zugabe von 30 Gew.-%iger NaOH konstant bei 8,5 (± 0,5) gehalten wurde. Anschließend wurde der pH durch Zugabe von 20 Gew.-%iger HCl auf 7,0 eingestellt und das Produkt mit Ethanol (92 Gew.-%) in einem Volumenverhältnis Lösung : Ethanol von 1 : 6 ausgefällt. Das Produkt wurde durch Abdekantieren der überstehenden Lösung isoliert und während 24 Stunden bei 50 °C und 125 mbar getrocknet.

Durch Variation der zugebenen Menge an Bernsteinsäureanhydrid wurden unterschiedliche Succinylierungsgrade erhalten. Die Ergebnisse sind in Tabelle 2 dargestellt.

**Tabelle 2**

| Beispiel | Äquivalente Bernsteinsäureanhydrid (bezogen auf Anhydroglucose) | Molarer Substitutionsgrad (¹H-NMR) | Ausbeute [%] (Mol isoliertes Produkt/Mol eingesetzte Anhydroglucose) |
|---|---|---|---|
| 8 | 0,17 | 0,15 | 74 |
| 9 | 0,08 | 0,07 | 82 |
| 10 | 0,04 | 0,03 | 84 |
| 11 | 0,02 | 0,02 | 70 |
| 1 | - | nicht derivatisiert | 84 |

Die Succinylierung beeinflusste die Löslichkelt des oxidierten Maltodextrins nicht signifikant.

Durch IR-Spektroskopie kann die Succinylierung durch Zunahme der Bande bei 1740 cm⁻¹ (C=O-Valenzschwingung von COOR/COOH) qualitataiv verfolgt werden. Der molare Succinylierungsgrad wurde durch ¹H-NMR-Spektroskopie durch das Verhältnis der Intensität der beiden CH₂-Signale bei 2,4 - 2,7 ppm (Succinylgruppe) zur Intensität des Signals bei 3,0 - 4,5 ppm und 5 - 6 ppm (7 Protonen der Anhydroglucosegruppe) bestimmt.

### Beispiele 12 bis 16

### Carboxymethylierung

200 g in Beispiel 1 erhaltenes C₁-oxidiertes Maltodextrin wurden in 660 ml Wasser gelöst. 118 g festes NaOH wurden zugegeben, so dass der pH 13-14 betrug. Chloressigsäure wurde portionsweise innerhalb 20 Minuten zugegeben und dann bei 25 °C während 3 Stunden gerührt. Anschließend wurde der pH durch Zugabe von 20 Gew.-%iger HCl auf 7,0 eingestellt und das Produkt mit Ethanol (92 Gew.-%) in einem Volumenverhältnis Lösung : Ethanol von 1 : 6 ausgefällt. Das Produkt wurde durch Abdekantieren der überstehenden Lösung isoliert und während 24 Stunden bei 50 °C und 125 mbar getrocknet.

Durch Variation der zugebenen Menge an Chloressigsäure wurden unterschiedliche Carboxymethylierungsgrade erhalten. Die Ergebnisse sind in Tabelle 3 dargestellt.

**Tabelle 3**

| Beispiel | Äquivalente Chloressigsäure (bezogen auf Anhydroglucose) | Molarer Substitutionsgrad (¹H-NMR) | Ausbeute [%] [Mol isoliertes Produkt/Mol eingesetzte Anhydroglucose) |
|---|---|---|---|
| 12 | 0,35 | 0,034 | 63 |
| 13 | 0,23 | 0,024 | 63 |
| 14 | 0,18 | 0,017 | 76 |
| 15 | 0,09 | 0,014 | 64 |
| 16 | 0,05 | 0,008 | 63 |
| 1 | - | nicht derivatisiert | 84 |

Die erreichten Carboxymethylierungsgrade beeinflussten die Löslichkeit des oxidierten Maltodextrins nicht signifikant.

Durch IR-Spektroskopie kann die Carboxymethylierung wegen der niedrigen Substitutionsgrade in diesen Beispielen nicht verfolgt werden. (Keine deutliche Bande bei 1 740 cm⁻¹ der C=O Valenzschwingung.) Der molare Carboxymethylierungsgrad wurde durch ¹H-NMR-Spektroskopie durch das Verhältnis der Intensität der anomeren Protonen bei 5,6 ppm (carboxymethylierte Anhydroglucosegruppe) zur Intensität des Signals der anomeren Protonen bei 4,8 - 5,8 ppm (Anhydroglucosegruppe ohne Derivatisierung) bestimmt.

### Beispiele 17 bis 20

### Sulfatierung

200 g in Beispiel 1 erhaltenes C₁-oxidiertes Maltodextrin wurden in 600 ml Wasser gelöst und auf 30 °C erwärmt. SO₃-Trimethylamin-Komplex wurde zugegeben und die Mischung bei 30 °C während 30 Minuten gerührt (die Suspension wandelte sich dabei in eine Lösung um). 40 Gew.-%ige NaOH (1.7 Äquivalente bezogen auf die molare Menge SO₃-Trimethylamin-Komplex, entsprechend 18 - 141 ml je nach Substitutionsgrad) wurde mit einer Rate von 2,8 ml/min zugegeben, und die Lösung wurde bei 30 °C während 2,5 Stunden gerührt. Der pH wurde mit 20 Gew.-%iger HCl auf 10,5 eingestellt. Das Produkt wurde mit 92 Gew.-%igem Ethanol ausgefällt in einem Volumenverhältnis Lösung : Ethanol von 1 : 7 - 1 : 8. Das Produkt wurde durch Abdekantieren der überstehenden Lösung isoliert und während 24 Stunden bei 50 °C und 1 25 mbar getrocknet.

Durch Variation der zugebenen Menge an SO₃-Triethylamin-Komplex wurden unterschiedliche Sulfatierungsgrade erhalten. Die Ergebnisse sind in Tabelle 4 dargestellt.

**Tabelle 4**

| Beispiel | Äquivalente SO₃-Reagenz (bezogen auf Anhydro-glucose) | Molarer Substitutionsgrad (¹H-NMR) | Ausbeute [%] (Mol isoliertes Produkt/Mol eingesetzte Anhydroglucose) |
|---|---|---|---|
| 17 | 0,67 | 0,56 | 98 |
| 18 | 0,34 | 0,27 | 92 |
| 19 | 0,17 | 0,12 | 93 |
| 20 | 0,08 | 0,05 | 86 |
| 1 | - | nicht derivatisiert | 84 |

Die steigende Ausbeute des oxidierten sulfatierten Maltodextrins ist durch die abnehmende Löslichkeit des Produkts in Ethanol begründet.

Durch IR-Spektroskopie kann der Sulfatierungsgrad qualitativ verfolgt werden (Zunahme der Banden bei 1260 und 830 cm⁻¹, Valenzschwingungen von SO₄².) Der molare Sulfatierungsgrad wurde durch ¹³C-NMR-Spektroskopie durch das Verhältnis der Intensität des C₁-Signals bei 96 ppm (sulfatierte Spezies) zur Intensität des C₁-Signals bei 103 ppm (nicht sulfatierte Spezies) bestimmt.

### Beispiele 21 bis 24

### Phosphatierung

300 g in Beispiel 1 erhaltenes C₁-oxidiertes Maltodextrin, NaH₂PO₄ und Na₂HPO₄ (molares Verhältnis 1 : 1,8) wurden in 7,5 I Wasser gelöst und der pH mit 20 Gew.-%iger HCl auf 3,0 eingestellt. Die Lösung wurde in einem Rotationsverdampfer bei 70 °C und 125 mbar zur Trockne eingedampft. Der Rückstand wurde während 16 Stunden bei 50 °C und 125 mbar getrocknet. Dieses Produkt wurde vermahlen und während 4 Stunden bei 750 mbar auf 160 °C erhitzt. Dieses Material wurde erneut vermahlen und in Wasser in einem Gewichtsverhältnis 1 : 4,4 (Feststoff : Wasser) bei 50 °C während 1 Stunde gelöst. Die Lösung wurde auf 25 °C gekühlt und die unlöslichen Rückstände wurden durch Zentrifugieren (5500 Upm während 1 Stunde) abgetrennt.

Die resultierende Lösung wurde zur Entfernung von freiem Orthophosphat durch Membranfiltration mit einer Nanofiltrationsmembran (Nitto-Denko NTR-7410, durchschnittlicher NaCl-Rückhalt 10 %) bei 22 bar und einer Flussrate von 180 - 210 I/Stunde filtriert. Die Entfernung des freien Orthophosphats wurde durch IR-Spektroskopie der Waschfraktionen kontrolliert. Die Lösung des oxidierten phosphatierten Maltodextrins wurde in einem Rotationsverdampfer bei 60 °C und 80 - 250 mbar auf 1 I konzentriert, und dann wurde das Produkt mit Ethanol in einem Volumenverhältnis 1 : 6 (Lösung : Ethanol) ausgefällt. Das Produkt wurde durch Zentrifugation der Suspension (5500 Upm für 1 Stunde) abgetrennt und während 24 Stunden bei 50 °C und 125 mbar getrocknet.

Durch Variation der zugebenen Menge des Gemisches von NaH₂PO₄ und Na₂HPO₄ in einem molaren Verhältnis von 1 : 1,8 wurden unterschiedliche Phosphatierungsgrade erhalten. Die Ergebnisse sind in Tabelle 5 dargestellt.

Der molare Substitutionsgrad wurde durch ICP-OES (Inductively Coupled Plasma-Optical Emission Spectroscopy, Gesamtphosphatgehalt) und lonenchromatographie gekoppelt mit Leitfähigkeitsmessung (Gehalt an freiem Monophosphat) bestimmt.

Eine qualitative Bestimmung des Gehalts an freiem Monophosphat wurde durch ³¹P-NMR-Spektroskopie durchgeführt. Polymer-gebundenes Monophosphat erscheint in Form breiter Signale im Bereich von ca. 0 - 2 ppm, während freies Monophosphat einen scharfen Peak bei ca. 0,7 ppm zeigt. Das breite Signal bei - 10 ppm kann Oligophosphaten zugeordnet werden.

**Tabelle 5**

| Beispiel | Äquivalente PO₄ (bezogen auf Anhydro-glucose) | Molarer Substitutionsgrad (ICP) | Freies PO₄ (ppm) | Freie Oligophosphate*** (ppm) | Ausbeute [%] (Mol isoliertes Produkt/Mol eingesetzte Anhydroglucose) |
|---|---|---|---|---|---|
| 21 | 1,85 | 0,25 | 80 | Nicht bestimmt | 22 |
| 22 | 0,55* | 0,08 | 1 | 22 | 22 |
| 23 | 0,28 | 0,24 | 2 | 55 | 13 |
| 24 | 0,23** | 0,08 | 58 | 52 | 18 |
| 1 | nicht derivatisiert | - | - | - | 84 |

| | | | | | |
|---|---|---|---|---|---|
| * Reaktionszeit bei 160 °C/740 mbar 16 Stunden statt 4 Stunden ** Die Maltodextrin/Phosphat-Lösung wurde mit Ethanol ausgefällt statt zur Trockne eingedampft *** Gehalt durch 31 P-NMR bestimmt | | | | | |

### Beispiele 25 bis 29

### C₂/C₃-Oxidation (zweistufige Synthese)

200 g in Beispiel 1 erhaltenes C1 -oxidiertes Maltodextrin wurden in 600 ml Wasser gelöst, und die Lösung wurde auf 50 °C erwärmt. Der pH wurde mit 20 Gew.-%iger HCl auf 8,5 bis 9,0 eingestellt, und 20 g NaOCl (14 bis 16 % aktives Chlor) wurden auf einmal zugegeben. Die restliche Menge NaOCl wurde mit einer Rate von 5,8 ml/min zugegeben, wobei der pH durch Zugabe von 30 Gew.-%iger NaOH konstant bei 8,5 (± 0,5) gehalten wurde. Die Lösung wurde während 1 Stunde bei 50 °C und pH 8,5 (± 0,5) gerührt. Der pH wurde anschließend mit 20 Gew.-%iger HCl auf 7 eingestellt. Das Produkt wurde mit 92 Gew.-%igem Ethanol ausgefällt in einem Volumenverhältnis Lösung : Ethanol von 1 : 6. Das Produkt wurde durch Abdekantieren der überstehenden Lösung isoliert und während 24 Stunden bei 50 °C und 125 mbar getrocknet.

### Beispiel 30

### C₁/C₂/C₃-Oxidation (einstufige Synthese, in-situ Derivatisierung)

200 g Maltodextrin mit einem Dextroseäquivalent von 12 wurden in 660 ml Wasser gelöst und die Lösung wurde auf 50 °C geheizt. 1,1 g NaBr wurden zugegeben und 135.2 g NaOCl-Lösung (14 bis 16 Gew.-% aktives Chlor) innerhalb von 30 Minuten zudosiert, wobei der pH bei 9,5 (±0,5) durch Zugabe von 30 Gew.-%iger NaOH konstant gehalten wurde. Die Lösung wurde während 1 Stunde bei 50 °C und pH 9,5 (± 0,5) gerührt. Der pH wurde anschließend mit 20 Gew.-%iger HCl auf 7 eingestellt. Das Produkt wurde mit 92 Gew.-%igem Ethanol ausgefällt in einem Volumenverhältnis Lösung : Ethanol von 1 : 6. Das Produkt wurde durch Abdekantieren der überstehenden Lösung isoliert und während 24 Stunden bei 50 °C und 125 mbar getrocknet.

Durch Variation der zugebenen Menge an NaOCl (14 - 16 % aktives Chlor) wurden unterschiedliche molare C₂/C₃-Oxidationsgrade erhalten. Die Ergebnisse sind in Tabelle 6 dargestellt.

**Tabelle 6**

| Beispiel | Äquivalente NaOCl | Molarer Oxidationsgrad (¹³C-NMR) | Ausbeute [%] (Mol isoliertes Produkt/Mol eingesetzte Anhydroglucose) |
|---|---|---|---|
| 25 | 0,48 | 0,042 | 72 |
| 26 | 0,24 | 0,022 | 71 |
| 27 | 0,12 | 0,012 | 88 |
| 28 | 0,06 | nicht nachweisbar | 75 |
| 29 | 0,03 | nicht nachweisbar | 78 |
| 30 | 0,12 | 0.017 | 89 |
| 1 | - | nicht derivatisiert | 84 |

Die Variation der isolierten Ausbeute der erhaltenen Produkte ist klein.

Durch IR-Spektroskopie konnte der Grad der C₂/C₃-Oxidation durch eine Zunahme der Bande bei 1640 cm⁻¹ verfolgt werden (C=O-Valenzschwingung von COO⁻).

Der molare Grad der C₂/C₃-Oxidation wurde durch ¹³C-NMR-Spektroskopie durch das Verhältnis der Intensität des COOH-Signals bei 1 75 und 176 ppm (oxidiertes C₂ und C₃) zur Intensität des Signals bei 76 - 84 ppm (nicht oxidiertes C₂) ermittelt.

### Allgemeine Arbeitsvorschrift 1 : Herstellung von Eisenkomplexen

Die Herstellung von Eisenkomplexen aus den erhaltenen oxidierten derivatisierten Maltodextrinen erfolgte jeweils mit 100 g des MaltodextrinDerivats:

Zu 352 g Eisen(III)-Chloridlösung (12 % Gew./Gew. Fe) wurden unter Rühren (Flügelrührer) bei Raumtemperatur zunächst 100 g des oxidierten derivatisierten Maltodextrins gelöst in 300 ml Wasser und dann 554 g Natriumcarbonatlösung (17.3 % Gew./Gew.) zugegeben.

Danach wurde durch Zugabe von Natronlauge ein pH von 1 1 eingestellt, die Lösung wurde auf 50 °C erwärmt und 30 Minuten bei 50 °C gehalten. Danach wurde durch Zugabe von Salzsäure auf einen pH von 5 bis 6 angesäuert, die Lösung wurde weitere 30 Minuten bei 50 °C gehalten und danach auf 97 - 98 °C erhitzt und 30 Minuten bei dieser Temperatur gehalten. Nach Abkühlen der Lösung auf Raumtemperatur wurde der pH-Wert durch Zusatz von Natronlauge auf 6 -7 eingestellt. Die Lösung wurde sodann über einen Sterilfilter filtriert und der Komplex wurde durch Ausfällen mit Ethanol im Verhältnis 1 : 0.85 isoliert und im Vakuum bei 50 °C getrocknet.

### Beispiele 31 bis 33

### Acetylierte Eisenkomplexe

Nach der allgemeinen Arbeitsvorschrift 1 wurden die acetylierten Eisenkomplexe 31 bis 33 aus den in Beispielen 5 bis 7 erhaltenen Maltodextrinderivaten erhalten, deren Eigenschaften in der folgenden Tabelle 7 zusammengefasst sind, jeweils verglichen mit einem Standardpräparat, das ebenfalls nach der allgemeinen Arbeitsvorschrift 1 aus C₁-oxidiertem, aber nicht derivatisiertem Maltodextrin wie in Beispiel 1 erhalten, hergestellt wurde.

**Tabelle 7**

| Parameter | Standard | Beispiel 31 MS = 0,14 (aus Beispiel 7) | Beispiel 32 MS = 0,31 (aus Beispiel 6) | Beispiel 33 MS = 0,61 (aus Beispiel 5) |
|---|---|---|---|---|
| Fe-Gehalt* | 27,0 | 28,9 | 29,7 | 30,6 |
| Mw | 168'000 | 234'000 | 349'000 | 511'000 |
| Mn | 100'000 | 139'000 | 163'000 | 334'000 |
| Abbaukinetik Θ = 0.5 | 35 | 41 | 46 | 44 |

| | | | | |
|---|---|---|---|---|
| *Werte bezogen auf Trockensubstanz. | | | | |

Die Verwendung von acetylierten Maltodextrinderivaten mit einem molaren Substitutionsgrad von >0,61 führte zu instabilen Produkten.

Die acetylierten Eisenkomplexe zeigten erhöhte Eisengehalte gegenüber dem Standard und ansteigende Molekulargewichte mit zunehmendem Substitutionsgrad. Die Abbaukinetik bei 50 % zeigte im Vergleich zum Standard ähnliche Werte. Die Fe-Ausbeuten der acetylierten Eisenkomlexe erreichten bis zu 97 %.

### Beispiele 34 bis 36

### Succinylierte Eisenkomplexe

Nach der allgemeinen Arbeitsvorschrift 1 wurden die succinylierten Eisenkomplexe 34 bis 36 aus den in Beispielen 9 bis 11 erhaltenen Maltodextrinderivaten erhalten, deren Eigenschaften in der folgenden Tabelle 8 zusammengefasst sind, jeweils verglichen mit einem Standardpräparat, das ebenfalls nach der allgemeinen Arbeitsvorschrift 1 aus C₁-oxidiertem, aber nicht derivatisiertem Maltodextrin wie in Beispiel 1 erhalten, hergestellt wurde.

**Tabelle 8**

| Parameter | Standard | Beispiel 34 MS = 0,02 (aus Beispiel 11) | Beispiel 35 MS = 0,03 (aus Beispiel 10) | Beispiel 36 MS = 0,07 (aus Beispiel 9) |
|---|---|---|---|---|
| Fe-Gehalt* | 27,0 | 24,3 | 26,9 | 24,4 |
| Mw | 168'000 | 260'000 | 347'000 | 773'000 |
| Mn | 100'000 | 128'000 | 145'000 | 188'000 |
| Abbaukinetik Θ = 0.5 | 35 | 28 | 32 | 6 |

| | | | | |
|---|---|---|---|---|
| * Werte bezogen auf Trockensubstanz. | | | | |

Die Verwendung von succinylierten Maltodextrinderivaten mit einem molaren Substitutionsgrad von >0,07 führte zu instabilen Produkten.

Die succinylierten Eisenkomplexe zeigten leicht reduzierte Eisengehalte gegenüber dem Standard sowie ansteigende Molekulargewichte mit zunehmendem Substitutionsgrad. Die Abbaukinetik bei 50 % zeigte im Vergleich zum Standard mit einer Ausnahme ähnliche Werte. Die Fe-Ausbeuten der succinylierten Komplexe erreichten bis zu 94 %.

### Beispiele 37 bis 38

### Carboxymethylierte Eisenkomplexe

Nach der allgemeinen Arbeitsvorschrift 1 wurden die carboxymethylierten Eisenkomplexe 37 und 38 aus den in Beispielen 15 und 16 erhaltenen Maltodextrinderivaten erhalten, deren Eigenschaften in der folgenden Tabelle 9 zusammengefasst sind, jeweils verglichen mit einem Standardpräparat, das ebenfalls nach der allgemeinen Arbeitsvorschrift 1 aus C₁-oxidiertem, aber nicht derivatisiertem Maltodextrin wie in Beispiel 1 erhalten, hergestellt wurde.

**Tabelle 9**

| Parameter | Standard | Beispiel 37 MS < 0,01 (aus Beispiel 16) | Beispiel 38 MS = 0,014 (aus Beispiel 15) |
|---|---|---|---|
| Fe-Gehalt* | 27,0 | 23,3 | 25,5 |
| Mw | 168'000 | 316'000 | 404'000 |
| Mn | 100'000 | 148'000 | 168'000 |
| Abbaukinetik Θ = 0.5 | 35 | 36 | 32 |

| | | | |
|---|---|---|---|
| *Werte bezogen auf Trockensubstanz. | | | |

Die Verwendung von carboxymethylierten Maltodextrinderivaten mit einem molaren Substitutionsgrad von >0,01 führte zu instabilen Produkten.

Die Eisengehalte der carboxymethylierten Eisenkomplexe zeigten sich leicht reduziert gegenüber dem Standard und die Molekulargewichte stiegen mit zunehmendem Substitutionsgrad an. Die Abbaukinetik bei 50 % zeigte im Vergleich zum Standard nahezu gleiche Werte. Die Fe-Ausbeuten der carboxymethylierten Eisenkomplexe erreichten bis zu 97 %.

### Beispiele 39 bis 41

### C₂/C₃-oxidierte Eisenkomplexe

Nach der allgemeinen Arbeitsvorschrift 1 wurden die C₂/C₃-oxidierten Eisenkomplexe 39 bis 41 aus den in Beispielen 27, 28 und 29 erhaltenen Maltodextrinderivaten erhalten, deren Eigenschaften in der folgenden Tabelle 10 zusammengefasst sind, jeweils verglichen mit einem Standardpräparat, das ebenfalls nach der allgemeinen Arbeitsvorschrift 1 aus C₁-oxidiertem, aber nicht derivatisiertem Maltodextrin wie in Beispiel 1 erhalten hergestellt wurde.

**Tabelle 10**

| Parameter | Standard | Beispiel 39 MS < 0,01 (aus Beispiel 29) | Beispiel 40 MS < 0,01 (aus Beispiel 28) | Beispiel 41 MS = 0,012 (aus Beispiel 27) |
|---|---|---|---|---|
| Fe-Gehalt* | 27.0 | 22.2 | 26.1 | 23.8 |
| Mw | 168'000 | 275'000 | 310'000 | 433'000 |
| Mn | 100'000 | 138'000 | 150'000 | 230'000 |
| Abbaukinetik Θ= 0.5 | 35 | 33 | 36 | 39 |

| | | | | |
|---|---|---|---|---|
| *Werte bezogen auf Trockensubstanz. | | | | |

Die Verwendung von C₂/C₃-oxidierten Maltodextrinderivaten mit einem molaren Substitutionsgrad von >0,01 führte zu instabilen Produkten.

Die Eisengehalte zeigten keine einheitliche Tendenz, die Molekulargewichte stiegen mit zunehmendem Substitutionsgrad an. Die Abbaukinetik bei 50 % zeigte im Vergleich zum Standard nahezu gleiche Werte. Die Fe-Ausbeuten der C2/C3-oxidierten Eisenkomplexe erreichten bis zu 95 %.

### Beispiele 42 bis 44

### Sulfatierte Eisenkomplexe (mehrstufige Synthese)

Nach der allgemeinen Arbeitsvorschrift 1 wurden die sulfatierten Eisenkomplexe 42 bis 44 in mehrstufigen Synthesen aus den in Beispielen 18 bis 20 erhaltenen Maltodextrinderivaten erhalten, deren Eigenschaften in der folgenden Tabelle 1 1 zusammengefasst sind, jeweils verglichen mit einem Standardpräparat, das ebenfalls nach der allgemeinen Arbeitsvorschrift 1 aus C₁-oxidiertem, aber nicht derivatisiertem Maltodextrin wie in Beispiel 1 erhalten, hergestellt wurde.

### Beispiel 45

### Sulfatierter Eisenkomplex (einstufige Synthese, in-situ Derivatisierung)

100 g Maltodextrin mit einem Dextroseäquivalent von 12 wurden in 300 ml Wasser gelöst. 0.7 g NaBr wurden zugegeben und 28.7 g NaOCl-Lösung (14 bis 16 Gew.-% aktives Chlor) wurden innerhalb von 30 Minuten zudosiert, wobei der pH bei 9,5 (±0,5) durch Zugabe von 30 Gew.-%iger NaOH konstant gehalten wurde. Die Lösung wurde anschließend auf 30 °C aufgeheizt, 14.4 g SO₃-Trimethylamin-Komplex zugegeben und 30 min bei 30 °C nachgerührt. Danach wurden 17.6 ml 40 Gew.-%ige NaOH zudosiert und 1 Stunde bei 30 °C nachgerührt.

Nach dem Abkühlen der Lösung auf 20 - 25 °C wurden unter Rühren 352 g Eisen(III)-Chloridlösung (12 % Gew./Gew. Fe) zugegeben und anschließend 554 g Natriumcarbonatlösung (17.3 % Gew./Gew.) zudosiert. Danach wurde durch Zugabe von Natronlauge ein pH von 11 eingestellt, die Lösung wurde auf 50 °C erwärmt und 30 Minuten bei 50 °C gehalten. Danach wurde durch Zugabe von Salzsäure auf einen pH von 5 bis 6 angesäuert, die Lösung wurde weitere 30 Minuten bei 50 °C gehalten und danach auf 97 - 98 °C erhitzt und 30 Minuten bei dieser Temperatur gehalten. Nach Abkühlen der Lösung auf Raumtemperatur wurde der pH-Wert durch Zusatz von Natronlauge auf 6 - 7 eingestellt. Die Lösung wurde sodann über einen Sterilfilter filtriert und der Komplex wurde durch Ausfällen mit Ethanol im Verhältnis 1 : 0.85 isoliert und im Vakuum bei 50 °C getrocknet.

**Tabelle 11**

| Parameter | Standard | Beispiel 42 MS = 0,05 (aus Beispiel 20) | Beispiel 43 MS = 0,12 (aus Beispiel 19) | Beispiel 44 MS = 0,27 (aus Beispiel 18) | Beispiel 45 MS = 0,12 |
|---|---|---|---|---|---|
| Fc-Gehalt* | 27,0* | 25,3 | 26,8 | 26,3 | 26,3 |
| Mw | 168'000 | 261'000 | 278'000 | 640'000 | 160'000 |
| Mn | 100'000 | 142'000 | 219'000 | 409'000 | 106'000 |
| Abbaukinetik Θ = 0.5 | 35 | 75 | 62 | 67 | - |

| | | | | | |
|---|---|---|---|---|---|
| * Werte bezogen auf Trockensubstanz. | | | | | |

Die Verwendung von sulfatierten Maltodextrinderivaten mit einem molaren Substitutionsgrad von >0,27 führte zu instabilen Produkten.

Die Eisengehalte der sulfatierten Eisenkomplexe blieben mit steigendem Substitutionsgrad nahezu konstant. Die Molekulargewichte der mehrstufig synthetisierten Eisenkomplexe stiegen mit zunehmendem Substitutionsgrad an. Die Abbaukinetik bei 50 % zeigte im Vergleich zum Standard erhöhte Werte. Die Fe-Ausbeuten der sulfatierten Eisenkomplexe erreichten bis zu 100 %.

## Patentansprüche

1. Wasserlöslicher Elsen-Kohlenhydratderivat-Komplex, erhältlich aus einer wässrigen Eisen(III)-Salzlösung und einer wässrigen Lösung des Produktes der Oxidation und anschließenden Derivatisierung von einem oder mehreren Maltodextrinen, wobei die Oxidation mit einer wässrigen Hypochloritlösung bei einem pH-Wert Im alkalischen Bereich durchgeführt wird, wobei beim Einsatz von einem Maltodextrin dessen Dextrose-Äquivalent bel 5 bis 20 und beim Einsatz eines Gemisches aus mehreren Maltodextrlnen das Dextrose-Äquivalent des Gemisches bei 5 bis 20 und das Dextrose Äquivalent der am Gemisch betelligten einzelnen Maltodextrine bel 2 bis 40 liegt, und die anschließende Derivatisierung mit einem geeigneten Reagenz erfolgt.

2. Wasserlöslicher Eisen-Kohlenhydrat-Komplex nach Anspruch 1. wobei die durch die Oxidation und Derivatisierung erhaltenen Maltodextrin-Derivate ausgewählt werden aus Estern monobasischer oder mehrbasischer Carbonsäuren, C₂/C₃-Oxidationsprodukten, Carboxyalkylierungsprodukten, Carbamaten, Ethern, Amiden, Anhydriden und Estern anorganischer Säuren,

3. Komplex nach Anspruch 1 oder 2, wobei die durch Oxidation und Derivatisierung erhaltenen Derivate von Maltodextrin ausgewählt werden aus Carbonsäureestern, gemischten Dicarbonsäureestern. Corboxyolkyllerungsprodukten, C₂/C₃-Oxidotionsprodukten, Phosphaten und Sulfaten.

4. Verfahren zur Herstellung eines Eisen-Kohlenhydrat-Komplexes nach einem oder mehreren der Ansprüche 1, **dadurch gekennzeichnet, dass** man ein oder mehrere Maltodextrine In wässeriger Lösung bei einem alkalischen pH-Wert mit einer wässerigen Hypochloritlösung oxidiert, die anschließende Derivatisierung mit einem geeigneten Reagenz durchführt und die erhaltene Lösung mit der wässerigen Lösung eines Elsen(III)-Salzes umsetzt, wobei beim Einsatz von einem Maltodextrin dessen Dextrose-Äquivalent bei 5 bis 20 und beim Einsatz eines Gemisches aus mehreren Maltodextrinen das Dextrose-Äquivalent des Gemisches bei 5 bis 20 und das Dextrose Äquivalent der am Gemisch beteiligten einzelnen Maltodextrine bei 2 bis 40 liegt.

5. Verfahren nach Anspruch 4, wobei die Derivatislerung des oxidierten Maltodextrins durch eines der folgenden Verfahren durchgeführt wird
a) Veresterung mit organischen oder anorganischen Säuren oder Derivaten davon
b) Oxidation
c) Carboxyalkylierung
d) Veretherung
e) Amidierung
f) Corbomatbildung
g) Anhydridbildung.

6. Verfahren nach Anspruch 4 oder 5 , wobei die Derlvatlslerung durch eines der folgenden Verfahren durchgeführt wird
a) Carboxyllerung mit monobasischen Carbonsäuren oder Carbonsäurederivaten
b) C₂/C₃-Oxidation
c) Carboxylierung mit dibasischen Carbonsäuren oder Garbonsäurederivaten
d) Carboxyalkylierung
e) Phosphatierung
f) Sulfatlerung,

7. Verfahren noch einem oder mehreren der Ansprüche 4 bis 6, **dadurch gekennzeichnet, dass** die Oxidation des Maltodextrins bzw. der Maltodextrine In Gegenwart von Bromidionen durchgeführt wird.

8. Verfahren nach einem oder mehreren der Ansprüche 4 bis 7, **dadurch gekennzeichnet, dass** als Elsen(III)-Salz Elsen(III)-Chlorid verwendet wird.

9. Verfahren nach einem oder mehreren der Ansprüche 4 bis 8, **dadurch gekennzeichnet, dass** oxidiertes, derivatisiertes Maltodextrin und Elsen(III)-Salz zu einer wässrigen Lösung mit einem pH-Wert, der so niedrig ist, dass keine Hydrolyse des Elsen(III)-Salzes auftritt, gemischt werden, worauf der pH-Wert durch Zusatz von Base auf 5 bis 1 2 angehoben wird,

10. Verfahren nach Anspruch 9, **dadurch gekennzeichnet, dass** man die Umsetzung 15 Minuten bis zu mehreren Stunden bei einer Temperatur von 15 ° C bis zum Siedepunkt durchführt.

11. Arzneimittel, enthaltend die wässrige Lösung eines Elsen-Kohlenhydratderivot-Komplexes gemäss Anspruch 1 bis 3, oder erhalten gemäss einem der Ansprüche 4 bis 10.

12. Arzneimittel gemäss Anspruch 11, **dadurch gekennzeichnet, dass** es zur parenteralen oder oralen Verabreichung formuliert ist.

13. Eisen-Kohlenhydratderivat-Komplex von Anspruch 1 bis 3, oder erhalten gemäss einem der Ansprüche 4 bis 10, zur Verwendung als Medikament.

14. Wasserlöslicher Eisen-Kohlenhydratderlvat-Komplex gemäss Anspruch 1 bis 3 zur Verwendung in einem Verfahren zur Behandlung oder Prophylaxe von Elsenmangelzuständen.

15. Verwendung der Elsen-Kohlenhydratderlvat-Komplexe von Anspruch 1 bis 3, oder erhalten gemäss einem der Ansprüche 4 bis 10, zur Herstellung eines Arzneimittels zur Behandlung oder Prophylaxe von Eisenmangelzuständen.

## Claims

1. Water-soluble iron-carbohydrate derivative complex obtainable from an aqueous iron(III) salt solution and an aqueous solution of the product of the oxidation and subsequent derivatization of one or more maltodextrins, wherein the oxidation is carried out with an aqueous hypochlorite solution at a pH value in the alkaline range, wherein, in the case of use of one maltodextrin its dextrose equivalent is from 5 to 20 and in the case of use of a mixture of a plurality of maltodextrins, the dextrose equivalent of the mixture is from 5 to 20 and the dextrose equivalent of each individual maltodextrin involved in the mixture is from 2 to 40, and the subsequent derivatization is effected with a suitable reagent.

2. Water-soluble iron-carbohydrate derivative complex of claim 1, wherein the maltodextrin derivatives obtained by oxidation and derivatization are selected from the group consisting of esters of monobasic or polybasic carboxylic acids, C₂/C₃ oxidized products, products of carboxyalkylation, carbamates, ethers, amides, anhydrides and esters of inorganic acids.

3. Complex of claim 1 or 2, wherein the derivatives of maltodextrin obtained by oxidation and derivatization are selected from the group consisting of esters of carboxylic acids, mixed esters of carboxylic acids, products of carboxyalkylation, C₂/C₃ oxidized products, phosphates and sulfates.

4. Process of preparation of an iron-carbohydrate complex according to one or more of claim 1 **characterized in that** one or more maltodextrins are oxidized in an aqueous solution with an aqueous hypochlorite solution at an alkaline pH value, the subsequent derivatization is performed with a suitable reagent and the obtained solution is reacted with an aqueous solution of an iron(III) salt, wherein, in the case of use of one maltodextrin its dextrose equivalent is from 5 to 20 and in the case of use of a mixture of a plurality of maltodextrins, the dextrose equivalent of the mixture is from 5 to 20 and the dextrose equivalent of each individual maltodextrin involved in the mixture is from 2 to 40.

5. Process of claim 4, wherein the derivatisation of the oxidized maltodextrins is performed by one of the following processes:
a) esterification with organic or inorganic acids or derivatives thereof
b) oxidation
c) carboxyalkylation
d) etherification
e) amidation
f) carbamate formation
g) anhydride formation.

6. Process of claim 4 or 5, wherein the derivatisation is performed by one of the following processes:
a) carboxylation with monobasic carboxylic acids or carboxylic acid derivatives
b) C₂/C₃ oxidation
c) carboxylation with dibasic carboxylic acids or carboxylic acid derivatives
d) carboxyalkylaion
e) posphatization
f) sulphation.

7. Process of one or more of claims 4 to 6 **characterized in that** the oxidation of the maltodextrin or maltodextrins is carried out in the presence of bromide ions.

8. Process of one or more of claims 4 to 7 **characterized in that** the iron (III) salt is iron (III) chloride.

9. Process of one or more of claims 4 to 8 **characterized in that** oxidized, derivatized maltodextrin and iron(III) salt are mixed into an aqueous solution having a pH value that is sufficiently low that hydrolysis of the iron(III) salt does not occur, whereupon the pH value is raised to from 5 to 12 by addition of a base.

10. Process of claim 9 **characterized in that** the reaction is carried out in the range of 15 minutes to several hours at a temperature in the range of 15°C to the boiling point.

11. Medicament comprising the aqueous solution of an iron-carbohydrate derivative complex according to any one of claims 1 to 3 or obtained according to any one of claims 4 to 10.

12. Medicament of claim 11 **characterized in that** it is formulated for parenteral or oral administration.

13. Iron-carbohydrate derivative complex of claim 1 to 3 or obtained according to one of claims 4 to 10 for use as a medicament.

14. Water-soluble iron-carbohydrate derivative complex of claims 1 to 3 for use in a process of treatment or prophylaxis of iron deficiency states.

15. Use of the iron-carbohydrate derivative complexes of claim 1 to 3 or obtained according to one of claims 4 to 10 for the preparation of a medicament for the treatment or prophylaxis of iron deficiency states.

## Revendications

1. Complexe de fer et de dérivés d'hydrates de carbone soluble dans l'eau pouvant être obtenu à partir d'une solution aqueuse de sel de fer(III) et d'une solution aqueuse du produit de l'oxydation et de la dérivatisation subséquente d'une ou plusieurs maltodextrines, dans lequel l'oxydation est réalisée avec une solution d'hypochlorite aqueuse à une valeur de pH dans la gamme alcaline, dans lequel quand une maltodextrine est utilisée son équivalent en dextrose est de 5 à 20 et quand un mélange d'une pluralité de maltodextrines est utilisé l'équivalent en dextrose du mélange est de 5 à 20 et l'équivalent en dextrose des maltodextrines individuelles dans le mélange est de 2 à 40, et la dérivatisation subséquente est réalisée avec un réactif approprié.

2. Complexe fer-hydrate de carbone soluble dans l'eau selon la revendication 1, dans lequel les dérivés de la maltodextrine obtenus par l'oxydation et la dérivatisation sont choisis parmi des esters d'acides carboxyliques monobasiques ou polybasiques, des produits d'oxydation de C₂/C₃, des produits de carboxyalkylation, des carbamates, des éthers, des amides, des anhydrides et des esters d'acides inorganiques.

3. Complexe selon la revendication 1 ou 2, dans lequel les dérivés de la maltodextrine obtenus par l'oxydation et la dérivatisation sont choisis parmi des esters d'acides carboxyliques, des esters d'acides dicarboxyliques mixtes, des produits de carboxyalkylation, des produits d'oxydation de C₂/C₃, des phosphates et des sulfates.

4. Procédé pour la préparation d'un complexe fer-hydrate de carbone selon l'une de la revendication 1, **caractérisé en ce qu'**une ou plusieurs maltodextrine(s) est/sont oxydée(s) en solution aqueuse, à une valeur de pH alcalin, avec une solution d'hypochlorite aqueuse, la dérivatisation subséquente est réalisée avec un réactif approprié, et la solution obtenue est mise à réagir avec la solution aqueuse d'un sel de fer(III), dans lequel quand une maltodextrine est utilisée son équivalent en dextrose est de 5 à 20 et quand un mélange d'une pluralité de maltodextrines est utilisé l'équivalent en dextrose du mélange est de 5 à 20 et l'équivalent en dextrose des maltodextrines individuelles dans le mélange est de 2 à 40.

5. Procédé selon la revendication 4, dans lequel la dérivatisation de la maltodextrine oxydée est réalisée par l'un des procédés suivants:
a) estérification avec des acides organiques ou inorganiques ou leurs dérivés,
b) oxydation,
c) carboxyalkylation,
d) éthérification,
e) amidation,
f) formation de carbamates,
g) formation d'anhydrides.

6. Procédé selon la revendication 4 ou 5, dans lequel la dérivatisation est réalisée par l'un des procédés suivants:
a) carboxylation avec des acides carboxyliques monobasiques ou des dérivés d'acide carboxylique,
b) oxydation de C₂/C₃
c) carboxylation avec des acides carboxyliques dibasiques ou des dérivés d'acide carboxylique,
d) carboxyalkylation,
e) phosphatation,
f) sulfatation.

7. Procédé selon une ou plusieurs des revendications 4 à 6, **caractérisé en ce que** l'oxydation de la maltodextrine ou des maltodextrines est réalisée en présence d'ions bromure.

8. Procédé selon l'une quelconque des revendications 4 à 7, **caractérisé en ce que** le sel de fer(III) utilisé est le chlorure de fer(III).

9. Procédé selon l'une ou plusieurs des revendications 4 à 8, **caractérisé en ce que** la maltodextrine oxydée, dérivatisée et le sel de fer(III) sont mélangés dans une solution aqueuse ayant une valeur de pH qui est suffisamment faible pour que l'hydrolyse du sel de fer(III) ne se produise pas, sur quoi la valeur du pH est élevée de 5 à 12 par addition de base.

10. Procédé selon la revendication 9, **caractérisé en ce que** la réaction est réalisée pendant 15 minutes à plusieurs heures à une température allant de 15 °C à la température d'ébullition.

11. Médicament comprenant la solution aqueuse d'un complexe de fer et de dérivés d'hydrates de carbone selon la revendication 1 à 3, ou pouvant être obtenu selon l'une quelconque des revendications 4 à 10.

12. Médicament selon la revendication 11, **caractérisé en ce qu'**il est formulé pour l'administration par voie parentérale ou orale.

13. Complexe de fer et de dérivés d'hydrates de carbone des revendications 1 à 3 ou obtenus selon l'une quelconque des revendications 4 à 10 pour le traitement ou la prophylaxie des états de déficit en fer.

14. Complexe de fer et de dérivés d'hydrates de carbone des revendications 1 à 3 à être utilisé dans un procédé pour le traitement ou la prophylaxie des états de déficit en fer.

15. Utilisation des complexes de fer et de dérivés d'hydrates de carbone soluble dans l'eau selon les revendications 1 à 3 ou obtenus selon l'une quelconque des revendications 4 à 10 pour la préparation d'un médicament pour le traitement ou la prophylaxie des états de déficit en fer.
